# NEW EUROPEAN PATENT SPECIFICATION

(11) **EP 0 321 176 B2**
(45) Date of publication and mention of the opposition decision: **24.03.2004**
(45) Mention of the grant of the patent: 08.02.1995
(21) Application number: 88311763.2
(22) Date of filing: 13.12.1988
(51) Int. Cl.: B29C 55/00, B29C 55/06, A61L 27/00

(54) **Process of forming a molecularly oriented thermoplastic member**
Verfahren zur Herstellung eines thermoplastischen Elementes mit molekularer Orienterung
Procédé pour la fabrication d'un élément thermoplastique à orientation moléculaire

(30) Priority: 14.12.1987 US 132755
(43) Date of publication of application: 21.06.1989
(73) Proprietor: JOHNSON & JOHNSON ORTHOPAEDICS INC., New Brunswick New Jersey 08903 (US)
(72) Inventor: Tunc, Deger, East Brunswick, NJ 08816 (US)
(74) Representative: Mercer, Christopher Paul

(56) References cited:
- EP-A- 0 202 090
- DD-A- 48 694
- GB-A- 1 123 445
- US-A- 3 531 561
- US-A- 3 797 499
- "Manufacture of Ultrahigh-Modules Poly(oxymethylenes) by Die Drawing" P.S. Hope, A. Richardson and I.M. Ward, Journal of Applied Polymer-Science, Vol. 26, 2879-2896 (1981)
- "Die-Drawing: Solid Phase Drawing of Polymers through a Converging Die" P.D. Coates & I.A. Ward, Polymer Engineering and Science, July 1981, Vol. 21, No. 10, pp. 612-618

## Description

### Background of the Invention

### Field of the Invention

The present invention relates to a process to orient a thermoplastic material which is absorbable in an animal body to increase its tensile strength.

### Prior Art

Polymers which are absorbable in an animal body have been used to manufacture absorbable sutures for some time. Internal bone fixation devices made with absorbable polymers have also been described. For example, U.S. Patents Nos. 4,539,981 and 4,550,449 describe absorbable bone fixation devices made from a high molecular weight of polymer of L(-)lactide. The polylactide bone fixation devices disclosed in the aforementioned patents can be used to fabricate plates and screws as well as intramedullary rods and pins which are used to secure the ends of fractured bones in proximity of each other so that they may properly heal.

Although the polylactide polymers are capable of being used for absorbable bone fixation devices it would be desirable if the absorbable polymer could be fabricated into plates, screws, pins and intramedullary rods which have even greater strength than the absorbable bone fixation devices described in the aforementioned patents.

Various techniques have been known for increasing the strength of thermoplastic materials. For example, it has been known for years to heat and stretch thermoplastic sheets and tubes to impart molecular orientation and thereby increase strength. U.S. Patents Nos. 3,248,463; 3,541,189; 3,630,824; 3,775,523; 4,157,235; 4,209,484; 4,413,110; and 4,577,998 teach such techniques.

### SUMMARY OF THE INVENTION

The present invention provides a process for increasing the tensile strength thermoplastic materials absorbable in an animal body, such as polyesters as set forth in claim 1. The process is particularly useful in treating of polylactide and similar polymers that are absorbable in an animal body. The process of the present invention provides significantly increased tensile strength and flexural strength for the absorbable pins, rods and screws which can be formed from this polymer. Generally, the process includes the steps of melting an absorbable polymer by molding with little or no draw and immediately cooling the polymer to a temperature below its glass transition temperature to cause nucleation and to form a self-supporting preformed member.

The term "self-supporting" as used herein describes a member having sufficient structural rigidity that if the member is gripped or supported at one end thereof, the opposite end thereof will remain essentially in the same plane as the gripped or supported end. The term "self-supporting" is intended to distinguish over flexible, or limber, members (such as a suture, monofilament fiber, or the like) which, if gripped or supported at one end, will bend or droop such that the end of the member opposite the gripped or supported end will be substantially below the plane of the gripped or supported end.

After the self-supporting preformed member has been initially cooled, it is then reheated to a temperature above its glass transition temperature and below its melting temperature while applying tension to the self-supporting preformed member during the reheating step. The member is then held under tension until it cools to room temperature. The application of tension is discontinued after the self-supporting member has cooled to permit the self-supporting member to relax. By processing the polymer in this manner, tensile strength increases of up to 800 percent can be achieved.

The drawings are described briefly as follows:
Figs. 1 and 1A are side elevational and cross-sectional views of a preformed rod member;
Figs. 2 and 2A are side elevational and cross-sectional views of a rod formed in accordance with the process of the present invention; and
Fig. 3 is a block diagram illustrating one form of the process of the present invention.

Referring to the drawings, the present invention provides a process to significantly improve the tensile and flexural strengths of large profile, rigid polymers. The term "large profile" is intended to include circular rods having a diameter in excess of 0.79mm (1/32 inch) as well as noncircular rods having dimensions of at least one side greater than 0.79 mm (1/32 inch). These materials are also rigid as compared, for example to a monofilament fiber of a similar diameter.

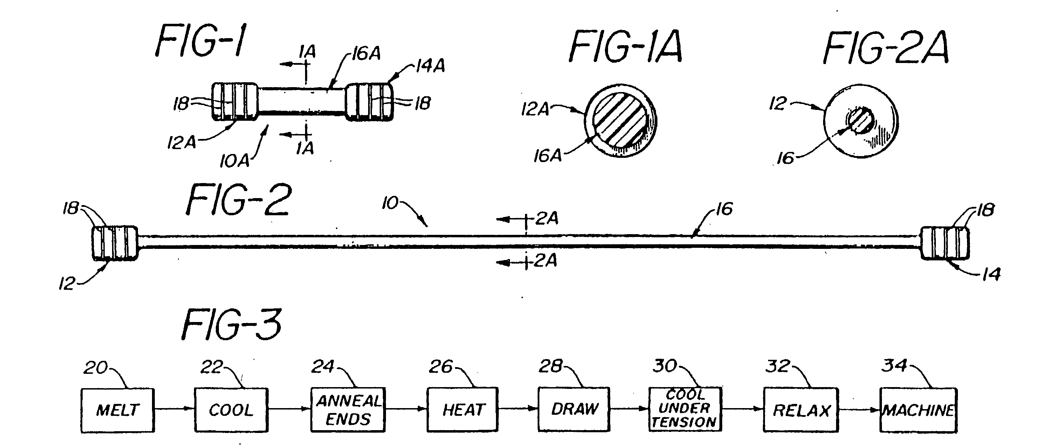

Referring now to Fig. 2, one form of a bone fixation device formed in accordance with the present invention is shown therein. Specifically, Fig. 2 illustrates a rod 10 having a first end portion 12, a second end portion 14, and a medial portion 16. Rod 10 is a solid, self-supporting member, and portions 12, 14 and 16 are circular in cross section (Fig. 2A). As will be evident from the ensuing description, the present invention contemplates that the formed, self-supporting member need not be solid, and may indeed be tubular. Furthermore, the formed, self-supporting member need not be circular in cross section, and may be oblong, triangular, hexagonal, or any other cross-sectional shape. Moreover, various different portions of any given self-supporting member may have different sizes and shapes.

In one specific form of the invention, the medial portion 16 of rod 10 has a length of 440 mm., and a diameter of 5.92 mm., while the end portions 12 and 14 have an average diameter of about 20 mm. End portions 12 and 14 are provided with a gripping means, such as grooves or ribs 18, to facilitate in holding the rod as it is drawn, from a preformed member, illustrated in Figs. 1 and 1A, to the configuration illustrated in Figs. 2 and 2A as described above.

With reference to Fig. 1, the preformed rod member 10A had an overall starting length of 98 mm., with a medial portion 16A having a length of 50 mm. and a diameter of 16 mm, and end portions 12A and 14A each having a length of 24 mm. End portions 12A and 14A had a starting diameter of 20 mm. As will become evident from the following description, during the process of manufacturing rod 10, tension is applied to preformed rod 10A to draw it to its final configuration.
It should be understood that the dimensions set forth above are for purposes of example only, and are not meant to impose any limitation on the invention, except to the extent that such dimensions may be incorporated in the appended claims. Moreover, the illustration and description of rod 10 is not meant to impose any limitation on the shape or size of any bone fixation device, or devices, by subsequent processing steps; such as cutting, shaping, boring, drilling, threading, etc.

The present invention contemplates that bone fixation devices of the type described above are formed by an intermittent process, e.g., injection molding. As will be appreciated, bone fixation devices formed by a process that includes a molding step could have a nonuniform shape, such as that illustrated in Figs. 1 and 2 wherein the end portions of the device are larger than the medial portion thereof.

The present invention specifically contemplates the bone fixation devices are formed of a polymer that is absorbable in an animal body, such as the polylactide polymers disclosed in U.S. Patents Nos. 4,539,981 and 4,550,449.

The melting point of such polylactide polymers varies depending on their thermal history. For example, if the polymer of the aforementioned patents is ground into a powder and fed directly into the cavity of an injection molding machine, it will melt at approximately 210°C. If the same polymer is melted and then formed into pellets, it will melt at a temperature of approximately 180°C. The highest temperature in the cavity of the injection molding machine may be just slightly higher, e.g., about 10°C higher, than the melting point of the polymer to avoid any degradation of the polymer.

Referring now to Fig. 3, an embodiment of the process of the present invention, which includes a molding step, is illustrated schematically therein. In the initial step 20 of the process, the polymer is melted by raising it to a temperature above its melting point, e.g. raised to a temperature of 210°C, and injected into the cavity of an injection molding machine, where it is formed into the desired shape.

The formed member is removed from the cavity, and immediately cooled, as shown schematically at 22, at a controlled rate to a temperature below the glass transition temperature of the polymer. It has been found that it is important to not cool the formed members too quickly to prevent, or at least substantial minimize, the formation of voids in the molded member. Likewise, it has been found that it is important to not cool the formed member too slowly to prevent, or at least substantially minimize, excessive crystallinity in the molded member. In accordance with a preferred embodiment of the present invention, immediately after the formed member is removed from the cavity of the injection molding machine, it is placed in an initial water bath at a temperature of about 65°C for a period of about 90 seconds, and thereafter placed in a secondary water bath at a temperature of about 42°C for a period of about 360 seconds. By quenching the formed member under these conditions, nucleation is promoted without excessive crystallinity and without the formation of voids. The cooled member will have the configuration shown in Fig. 1.

As an optional, but preferred, next step, the ends 12A and 14A of the formed and cooled member are annealed as shown schematically at 24 in Fig. 3. The annealing step may be performed, for example, by immersing the ends of the rod 10A in a glycerin bath at a temperature of about 100°C for a period of 10 minutes. It is important that the rod 10A not be retained in the annealing bath for too long of a period of time to prevent the medial portion 16A of the rod from crystallizing. It will be understood that the annealing step may be performed by inserting only the ends 12A and 14A in the annealing bath.

After the formed member has been cooled, and the ends optionally annealed, the formed member is reheated to a temperature above the glass transition temperature of the polymer, but below its melting temperature, as is shown schematically at 26 in Fig. 3. As will hereinafter be described, tension is applied to the formed member during at least a portion of the reheating step to molecularly orient the medial portion of the formed member. To this end, the ends 12A and 14A are gripped in the jaws of a conventional tnstron testing machine, and a heated liquid is circulated throughout the interior of a chamber positioned between the jaws of the Instron machine to substantially uniformly heat the rod 10A to the desired temperature.

In accordance with one preferred embodiment of the invention, the heating liquid is water, although it should be understood that other inert liquids or gases are within the contemplation of the present invention, provided that adequate heat can be transferred to the rod 10A. The water is heated in a separate reservoir to a temperature of about 98°C and is introduced into the Instron tester at that temperature. The water exits the Instron tester at a temperature of about 90°C, and is effective to heat the rod 10A substantially uniformly to a temperature of about 87°C in about 3 minutes.

As the rod 10A is heated, it is subjected to solid state deformation. In this regard, the jaws of the Instron tester are moved relative to one another to apply tension to the medial portion of the rod, as is shown schematically at 28 in Fig. 3. It is important to control the speed of jaw movement in order to prevent breakage, and yet get the desired degree of molecular orientation before excessive recrystallization occurs. In accordance with one preferred embodiment of the invention the gripping jaws are initially moved apart at a rate of about 50.8 cm (20 inches) per minute until a load of about 113-118 kg (250-260 pounds) is applied to the rod. The pulling speed is then reduced to about 25.4 cm (10 inches) per minute, and is maintained at that rate until a load of about 136 kg (300 pounds) is applied to the rod. Thereafter the pulling speed is reduced to about 12.7 cm (5 inches) per minute and is maintained until a load of about 159 kg (350 pounds) is applied to the rod. Subsequently the pulling speed is gradually reduced to zero, when the desired length is obtained while continuing to maintain a load of about 159 kg (350 pounds) on the rod.

In accordance with one very important aspect of the present invention, once the rod has reached its final drawn length, the applied tension is retained. The heating liquid is then quickly drained from Instron tester, and cooling liquid is immediately circulated through the Instron tester as is shown schematically at 30 in Fig. 3. While other cooling fluids may be used, it has been found that circulating ice water at a temperature of about 6°C to about 15°C for a period of about 5 minutes has worked extremely well in quickly and uniformly cooling the drawn rod to the desired temperature. It is thought that the shrink energy of the rod during the cooling under tension step contributes significantly to the increase in ultimate tensile strength of the resulting product.

After the rod has been cooled, the tension is released and the rod is permitted to relax, as is shown schematically at 32 in Fig. 3. Thereafter the rod may be machined, as shown schematically at 34 in Fig. 3, as by cutting off ends 12 and 14, cutting medial portion 16 into shorter lengths, threading, etc.

### EXAMPLES

### EXAMPLE 1 (NOT ACCORDING TO INVENTION)

This example illustrates the preparation of an unoriented control sample. Pelletized, high molecular weight polylactide is extruded through a commercial extruder. No tension is applied to the polylactide rod as it exits the extruder nozzle. The polylactide rod is quenched in a water spray and then is passed through an 8 foot long water bath maintained at a temperature of 60°C in a relaxed state. This corresponds to a draw ratio of 1. The sample was then tested and had the following properties:

| | |
|---|---|
| Ultimate tensile strength, mpa | 22.8 |
| Secant modulus, mpa | 1564 |
| Elongation percent | 4 |

### EXAMPLE 2 (NOT ACCORDING TO INVENTION)

This example illustrates the effect of applying tension to the polylactide rod after it is extruded, but before it is quenched.

High molecular weight polylactide pellets as used in Example 1 were extruded under the same conditions as set forth in Example 1. Tension was applied to the polylactide rod as it came out of the nozzle of the extruder. The draw ratio was 4.4:1. The material was cooled to room temperature without passing through the 60°C bath as in Example 1. The product was tested for tensile strengths and had the following properties:

| | |
|---|---|
| Ultimate tensile strength, mpa | 25.5 |
| Secant modulus, mpa | 1205 |
| Elongation percent | 22 |

This sample did not have a significantly different tensile strength than the sample of Example 1.

### EXAMPLE 3 (NOT ACCORDING TO INVENTION)

This example illustrates the effect of a hot water heating treatment after deformation without a nucleation or quenching step.

The polylactide pellets used in Example 1 were extruded at a draw ratio to 4.4:1 as in Example 2. The extruded polylactide was heat-treated by passing it through a heating tank containing a water bath at 60°C in a relaxed state. Tensile tests were performed with a sample with the following results:

| | |
|---|---|
| Ultimate tensile strength, mpa | 68.6 |
| Secant modulus, mpa | 1539 |
| Elongation percent | 11 |

### EXAMPLE 4 (NOT ACCORDING TO INVENTION)

Polylactide pellets of the same type used in Example 1 were extruded through a 19 mm (0.75 inch) diameter die. The extruded rod was immediately quenched by spraying cold water of a temperature of approximately 20°C while the rod was pulled to a diameter of 12.2 mm (0.48 inches). This corresponds to a draw ratio of 2.4:1. The polylactide rod was reheated to a temperature immediately above its glass transition temperature. During the heating process the rod was subjected to a further draw with a draw ratio of 4.0:1. The sample was cooled while it was kept under tension. Once the rod was cooled to room temperature, the tension was relaxed. The tensile test results of this sample were as follows:

| | |
|---|---|
| Ultimate tensile strength, mpa | 187 |
| Secant modulus, mpa | 7409 |
| Elongation percent | 82 |

By comparing Example 1 to Example 2 it is evident that while the application of strain prior to nucleation or quenching does not significantly improve the tensile strength, it does significantly increase the elongation value. By comparing Example 3 to Example 2 it is evident that while the solid state deformation without nucleation does not help the strength significantly, the subsequent heat treatment improves both the strength and the modulus.

### EXAMPLE 5 (ACCORDING TO INVENTION)

This example illustrates the present invention when the preformed member is obtained by injection molding.

Further injection molded polylactide samples were prepared in accordance with the process described before in connection with Figs. 1, 2 and 3, and evaluated to determine the effect that various draw ratios had on ultimate tensile strength. The results of this evaluation are set forth in the following table:

| Sample | Draw ratio | Ultimate tensile strength (mpa) |
|---|---|---|
| Control | 1 | 62 |
| 1 | 2.63 | 90 |
| 2 | 3.13 | 141 |
| 3 | 3.75 | 223 |
| 4 | 4.65 | 245 |
| 5 | 5.35 | 294 |
| 6 | 6.34 | 317 |
| 7 | 6.46 | 279 |
| 8 | 6.8 | 295 |
| 9 | 6.82 | 296 |
| 10 | 722 | 309 |
| 11 | 7.82 | 326 |
| 12 | 7.96 | 335 |
| 13 | 8.4 | 351 |
| 14 | 8.82 | 338 |
| 15 | 10.61 | 492 |

From the above, it is clear that, in accordance with the present invention, as the draw ratio increases there is a direct and substantially proportionate increase in ultimate tensile strength.

## Claims

1. A process of forming a self-supporting, high strength, thermoplastic member having a minimum cross-sectional dimension of at least 0.79 mm, comprising the steps of
providing a thermoplastic polymer capable of being absorbed in an animal body; melting the thermoplastic polymer and forming the melted polymer into a preformed member by an intermittent molding process;
cooling the preformed member to a temperature below the glass transition temperature of the thermoplastic polymer to cause nucleation and to cause the preformed member to become self-supporting;
reheating the self-supporting, preformed member to a temperature above the glass transition temperature, but below the melting temperature, of the thermoplastic polymer;
drawing the reheated self-supporting preformed member under tension during said reheating step;
cooling the drawn reheated self-supporting preformed member while maintaining the tension applied thereto; and
discontinuing the application of tension after the drawn reheated self-supporting preformed member has cooled to permit it to relax,
thereby providing said self-supporting, high strength, thermoplastic member.

2. The process of claim 1, wherein the initial cooling step is performed by exposing the preformed member to a cooling fluid.

3. The process of claim 2, wherein said cooling fluid is air.

4. The process of claim 2, wherein said cooling fluid is water.

5. The process of claim 4, wherein the water is applied by spraying.

6. The process of claim 4, wherein the preformed member is cooled by immersing it in a water bath.

7. The process of any one of claims 1 to 6, wherein said thermoplastic polymer is melted by processing it in an injection molder.

8. The process of claim 7, wherein the thermoplastic polymer is molded into the shape of a rod.

9. The process of claim 8, wherein the ends of said rod are annealed before said reheating step.

10. The process of any one of claims 1 to 9, wherein said preformed member is preheated during said reheating step prior to the drawing under tension.

11. The process of any one of claims 1 to 10, wherein the step of applying tension is performed by effecting relative longitudinal movement between two spaced points on said member at a given rate until a predetermined load is applied, and then gradually reducing the rate of said relative movement.

12. The process of any one of claims 1 to 11, wherein the final cooling step is performed by exposing the member to a cooling fluid.

13. The process of claim 12, wherein the cooling fluid is water.

14. The process of any one of the preceding claims, wherein the thermoplastic polymer is a polylactide polymer.

15. The process of claim 14, wherein the preformed member is drawn under tension at a controlled rate at a draw ratio of from 2 to 12 times the length of the preformed member.

## Patentansprüche

1. Verfahren zum Ausbilden eines freitragenden,hochfesten, thermoplastischen Elements mit einer Mindestquerschnittsabmessung von mindestens 0.79 mm, umfassend die Schritte:
Bereitstellen eines thermoplastischen Polymers, das in einem Tierkörper resorbiert werden kann;
Schmelzen des thermoplastischen Polymers und Formen des geschmolzenen Polymers zu einem vorgeformten Element mittels eines diskontinuierlichen Gießverfahrens; Kühlen des vorgeformten Elementes auf eine Temperatur unterhalb der Glasübergangstemperatur des thermoplastischen Polymers, um Keimbildung zu bewirken und um das vorgeformte Element freitragend zu machen;
Wiedererwärmen des freitragenden, vorgeformten Elementes auf eine Temperatur oberhalb der Glasübergangstemperatur des thermoplastischen Polymers, jedoch unterhalb der Schmelztemperatur des thermoplastischen Polymers;
Anlegen einer Zugspannung an das wiedererwärmte, freitragende, vorgeformte Element während des Wiedererwärmungsschrittes;
Kühlen des gezogenen, wiedererwärmten, freitragenden, vorgeformten Elements, während die daran angelegte Spannung aufrechterhalten wird; und
Unterbrechen der angelegten Zugspannung, nachdem das gezogene, wiedererwärmte, freitragende, vorgeformte Element abgekühlt ist, damit es sich entspannt,
wodurch das freitragende, hochfeste, thermoplastische Element entsteht.

2. Verfahren nach Anspruch 1, wobei der erste Kühlschritt durchgeführt wird, indem das Element einem kühlenden Fluid ausgesetzt wird.

3. Verfahren nach Anspruch 2, wobei das kühlende Fluid Luft ist.

4. Verfahren nach Anspruch 2, wobei das kühlende Fluid Wasser ist.

5. Verfahren nach Anspruch 4, wobei das Wasser durch Sprühen aufgebracht wird.

6. Verfahren nach Anspruch 4, wobei das vorgeformte Element durch Eintauchen in ein Wasserbad gekühlt wird.

7. Verfahren nach irgendeinem der Ansprüche 1 bis 6, wobei das thermoplastische Polymer durch Verarbeiten in einer Spritzgießvorrichtung geschmolzen wird.

8. Verfahren nach Anspruch 7, wobei das thermoplastische Polymer in die Form eines Stabes gebracht wird.

9. Verfahren nach Anspruch 8, wobei die Enden des Stabes vor dem Wiedererwärmungsschritt gekühlt werden.

10. Verfahren nach einem der Ansprüche 1 bis 9, wobei das vorgeformte Element während des Wiedererwärmungsschritts vor Anlegen der Zugspannung vorgewärmt wird.

11. Verfahren nach einem der Ansprüche 1 bis 10, wobei der Schritt des Aufbringens der Zugspannung durch eine relative Längsbewegung zwischen zwei räumlich getrennten Stellen an dem Element bei einer gegebenen Geschwindigkeit bewirkt wird, bis eine vorbestimmte Spannung erreicht ist, und dann die Geschwindigkeit der relativen Bewegung allmählich reduziert wird.

12. Verfahren nach einem der Ansprüche 1 bis 11, wobei der letzte Kühlschritt dadurch ausgeführt wird, dass das Element einem kühlenden Fluid ausgesetzt wird.

13. Verfahren nach Anspruch 12, wobei das kühlende Fluid Wasser ist.

14. Verfahren nach irgendeinem der vorhergehenden Ansprüche, wobei das thermoplastische Polymer ein Polylactidpolymer ist.

15. Verfahren nach Anspruch 14, wobei das vorgeformte Element unter Spannung bei einem kontrollierten Reckverhältnis des 2- bis 12-fachen der Länge des vorgeformten Elementes gezogen wird.

## Revendications

1. Procédé pour former un élément thermoplastique, hautement résistant, auto-portant, ayant une dimension de section minimale d'au moins 0,79 mm, comprenant les étapes consistant à :
- fournir un polymère thermoplastique résorbable par l'organisme d'un animal ;
- fondre le polymère thermoplastique et former le polymère fondu en un élément préformé par un procédé de moulage intermittent ;
- refroidir l'élément préformé à une température inférieure à la température de transition vitreuse du polymère thermoplastique afin de provoquer une nucléation et à rendre l'élément préformé auto-portant ;
- réchauffer l'élément préformé, auto-portant à une température supérieure à la température de transition vitreuse, mais inférieure à la température de fusion du polymère thermoplastique ;
- étirer l'élément préformé, auto-portant, réchauffé, sous traction pendant ladite étape de réchauffage ;
- refroidir l'élément préformé, auto-portant, réchauffé, étiré, tout en maintenant la traction appliquée à celui-ci ; et
- interrompre l'application de traction après que l'élément préformé, auto-portant, réchauffé, étiré, ait refroidi afin de permettre sa relaxation ;
- fournir ainsi ledit élément thermoplastique, hautement résistant, auto-portant.

2. Procédé selon la revendication 1, dans lequel l'étape de refroidissement initiale est réalisée en exposant l'élément préformé à un fluide de refroidissement.

3. Procédé selon la revendication 2, dans lequel ledit fluide de refroidissement est l'air.

4. Procédé selon la revendication 2, dans lequel ledit fluide de refroidissement est l'eau.

5. Procédé selon la revendication 4, dans lequel l'eau est appliquée par pulvérisation.

6. Procédé selon la revendication 4, dans lequel l'élément préformé est refroidi en l'immergeant dans un bain d'eau.

7. Procédé selon l'une quelconque des revendications 1 à 6, dans lequel la fusion dudit polymère thermoplastique est réalisée par traitement dans une machine à mouler par injection.

8. Procédé selon la revendication 7, dans lequel le polymère thermoplastique est moulé en forme de tige.

9. Procédé selon la revendication 8, dans lequel les extrémités de ladite tige sont recuites avant ladite étape de réchauffage.

10. Procédé selon l'une quelconque des revendications 1 à 9, dans lequel ledit élément préformé est préchauffé pendant ladite étape de réchauffage avant l'étirage sous traction.

11. Procédé selon l'une quelconque des revendications 1 à 10, dans lequel l'étape consistant à appliquer une traction est réalisée en effectuant un mouvement longitudinal relatif entre deux points espacés sur ledit élément à une vitesse donnée jusqu'à ce qu'une charge prédéterminée soit appliquée, puis en réduisant progressivement la vitesse dudit mouvement relatif.

12. Procédé selon l'une quelconque des revendications 1 à 11, dans lequel l'étape de refroidissement finale est réalisée en exposant l'élément à un fluide de refroidissement.

13. Procédé selon la revendication 12, dans lequel le fluide de refroidissement est l'eau.

14. Procédé selon l'une quelconque des revendications précédentes, dans lequel le polymère thermoplastique est un polymère polylactide.

15. Procédé selon la revendication 14, dans lequel l'élément préformé est étiré sous traction à une vitesse régulée à un rapport d'étirement de 2 à 12 fois la longueur de l'élément préformé.
